(19) Europäisches Patentamt
European Patent Office
Office européen des brevets

(11) Veröffentlichungsnummer: **0 327 913 B1**

# (12) EUROPÄISCHE PATENTSCHRIFT

(45) Veröffentlichungstag der Patentschrift: **06.04.94**

(51) Int. Cl.5: **C07D 233/60**, C07D 249/08, A01N 43/50, A01N 43/653, C07D 405/06

(21) Anmeldenummer: **89101521.6**

(22) Anmeldetag: **30.01.89**

Die Akte enthält technische Angaben, die nach dem Eingang der Anmeldung eingereicht wurden und die nicht in dieser Patentschrift enthalten sind.

## (54) Substituierte Azolylmethyloxirane.

(30) Priorität: **09.02.88 DE 3803833**

(43) Veröffentlichungstag der Anmeldung: **16.08.89 Patentblatt 89/33**

(45) Bekanntmachung des Hinweises auf die Patenterteilung: **06.04.94 Patentblatt 94/14**

(84) Benannte Vertragsstaaten: **BE CH DE FR GB IT LI NL**

(56) Entgegenhaltungen:
**EP-A- 0 094 564**

**JOURNAL OF HETEROCYCLIC CHEMISTRY, September-Oktober 1988, Seiten 1439-1441; M.MORENO-MANAS et al.: "Synthesis of 2,3-diaryl-1,4-diazolyl-2,3-epoxybutanes"**

(73) Patentinhaber: **BAYER AG**
**D-51368 Leverkusen(DE)**

(72) Erfinder: **Elbe, Hans-Ludwig, Dr.**
**Dasnöckel 59**
**D-5600 Wuppertal 11(DE)**
Erfinder: **Büchel, Karl-Heinz, Prof. Dr.**
**Dabringhausener Strasse 42**
**D-5093 Burscheid(DE)**
Erfinder: **Brandes, Wilhelm, Dr.**
**Eichendorffstrasse 3**
**D-5653 Leichlingen(DE)**
Erfinder: **Dutzmann, Stefan, Dr.**
**Leinenweberweg 33**
**D-4000 Düsseldorf 13(DE)**
Erfinder: **Lürssen, Klaus, Dr.**
**August-Kierspel-Strasse 145**
**D-5060 Bergisch-Gladbach 2(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue substituierte Azolylmethyloxirane, ein Verfahren zu ihrer Herstellung und ihre Verwendung als Fungizide und Pflanzenwachstumsregulatoren.

Es ist bereits bekannt geworden, daß bestimmte Hydroxyalkylazole fungizide Eigenschaften aufweisen (vergleiche DE- A 32 45 504). So können z.B. 3-(4-Chlor-phenylthio)-2-(4-chlor-phenyl)-3,3-dimethyl-1-(imidazol-1-yl)-2-propanol, 3-(2,4-Dichlor-phenoxy)-2-(4-chlor-phenyl)-3,3-dimethyl-1-(imidazol-1-yl)-2-propanol und 2-(4-Chlorphenyl)-4-(4-fluorphenyl)-3,3-dimethyl-1-(1,2,4-triazol-1-yl)-2-butanol zur Bekämpfung von Pilzen eingesetzt werden. Die Wirksamkeit dieser Stoffe ist jedoch, vor allem bei niedrigen Aufwandmengen und -konzentrationen, nicht immer ganz befriedigend.

Außerdem sind aus der EP-A 0 094 564 fungizid wirksame Azolylmethyloxirane bekannt, in denen der Oxiran-Ring dreifach substituiert ist. Entsprechende Oxirane mit vier Substituenten werden jedoch nicht beschrieben.

Es wurden nun neue substituierte Azolylmethyloxirane der Formel

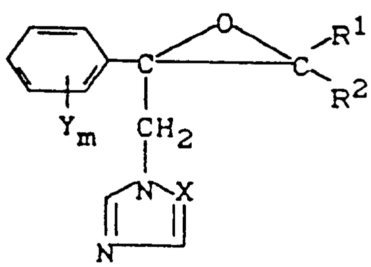

(I)

in welcher

- $R^1$ für geradkettiges oder verzweigtes Alkyl mit 1 bis 4 Kohlenstoffatomen steht,
- $R^2$ für geradkettiges oder verzweigtes Alkyl mit 1 bis 4 Kohlenstoffatomen steht,
- X für ein Stickstoffatom oder die CH-Gruppe steht,
- Y für Halogen, Alkyl mit 1 bis 4 Kohlenstoffatomen, Cycloalkyl mit 3 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, sowie für gegebenenfalls durch Halogen und/oder Alkyl mit 1 oder 2 Kohlenstoffatomen substituiertes Phenyl, gegebenenfalls durch Halogen und/oder Alkyl mit 1 oder 2 Kohlenstoffatomen substituiertes Phenoxy, gegebenenfalls durch Halogen und/oder Alkyl mit 1 oder 2 Kohlenstoffatomen substituiertes Phenylalkyl mit 1 oder 2 Kohlenstoffatomen im Alkylteil und/oder für gegebenenfalls durch Halogen und/oder Alkyl mit 1 oder 2 Kohlenstoffatomen substituiertes Phenylalkoxy mit 1 oder 2 Kohlenstoffatomen im Alkoxyteil steht, und
- m für die Zahlen 0, 1, 2 oder 3 steht,

wobei die Bedeutungen von Y gleich oder verschieden sein können, wenn m für die Zahlen 2 oder 3 steht, sowie deren Säureadditions-Salze und Metallsalz-Komplexe gefunden.

Die neuen substituierten Azolylmethyloxirane der Formel (I) besitzen für den Fall, daß $R^1$ und $R^2$ voneinander verschieden sind, zwei benachbarte asymmetrisch substituierte Kohlenstoffatome. Sie können deshalb in den beiden geometrischen Isomeren (threo- und erythro-Form) vorliegen, die in unterschiedlichen Mengenverhältnissen anfallen können. Die Erfindung betrifft sowohl die einzelnen Isomeren als auch deren Gemische.

Weiterhin wurde gefunden, daß man substituierte Azolylmethyloxirane der Formel (I) bzw. deren Säureadditions-Salze und Metallsalz-Komplexe erhält, wenn man Hydroxyalkylazole der Formel

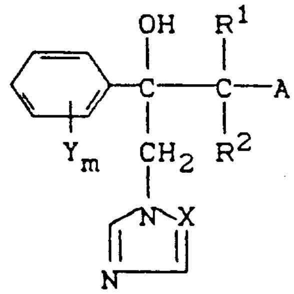

(II)

in welcher

| | |
|---|---|
| $R^1$, $R^2$, X, Y und m | die oben angegebene Bedeutung haben und |
| A | für einen Phenolat-, 4-Chlorphenolat- oder Thiophenolat-Rest steht, |

gegebenenfalls in Gegenwart eines Verdünnungsmittels mit starken Basen umsetzt und gegebenenfalls anschließend an die so erhaltenen Verbindungen der Formel (I) eine Säure oder ein Metallsalz addiert.

Schließlich wurde gefunden, daß die neuen substituierten Azolylmethyloxirane der Formel (I) sowie deren Säureadditions-Salze und Metallsalz-Komplexe sehr gute fungizide und pflanzenwachstumsregulierende Eigenschaften besitzen.

Überraschenderweise zeichnen sich die erfindungsgemäßen Stoffe durch eine bessere fungizide Wirkung aus als ähnliche, aus dem Stand der Technik bekannte Verbindungen gleicher Indikation. So übertreffen die erfindungsgemäßen Stoffe zum Beispiel 3-(4-Chlor-phenylthio)-2-(4-chlor-phenyl)-3,3-dimethyl-1-(imidazol-1-yl)-2-propanol, 3-(2,4-Dichlor-phenoxy)-2-(4-chlor-phenyl)-3,3-diemthyl-1-(imidazol-1-yl)-2-propanol und 2-(4-Chlorphenyl)-4-(4-fluorphenyl)-3,3-dimethyl-1-(1,2,4-triazol-1-yl)-2-butanol bezüglich ihrer fungiziden Eigenschaften.

Außerdem sind die neuen substituierten Azolylmethyloxirane der Formel (I) interessante Zwischenprodukte zur Herstellung von weiteren Pflanzenschutz-Wirkstoffen. So können durch entsprechende Umsetzungen der Oxirane mit Triazolen oder Imidazolen Fungizide und Antimykotika erhalten werden (EP-A 0 131 845 und DE-A 33 07 218).

Weiterhin können durch Umsetzung der Oxirane mit einem Sauerstoff-oder Schwefel-Nucleophil bekannte Fungizide erhalten werden (vgl. EP-A 0 061 835).

Die erfindungsgemäßen substituierten Azolylmethyloxirane sind durch die Formel (I) allgemein definiert. Bevorzugt sind Verbindungen der Formel (I), in denen

| | |
|---|---|
| $R^1$ | für Methyl oder Ethyl steht, |
| $R^2$ | für Methyl oder Ethyl steht, |
| X | für ein Stickstoffatom oder die CH-Gruppe steht, |
| Y | für Fluor, Chlor, Brom, Methyl, Isopropyl, t-Butyl, Cyclohexyl, Methoxy, Methylthio, Trifluormethyl, Trifluormethoxy, Trifluormethylthio sowie für gegebenenfalls durch Fluor, Chlor und/oder Methyl substituiertes Phenyl, gegebenenfalls durch Fluor, Chlor und/oder Methyl substituiertes Phenoxy, gegebenenfalls durch Fluor, Chlor und/oder Methyl substituiertes Benzyl und/oder für gegebenenfalls durch Fluor, Chlor und/oder Methyl substituiertes Benzyloxy steht und |
| m | für die Zahlen 0, 1, 2 oder 3 steht. |

Bevorzugte erfindungsgemäße Verbindungen sind auch Additionsprodukte aus Säuren und denjenigen substituierten Azolylmethyloxiranen der Formel (I), in denen die Substituenten $R^1$, $R^2$, X , Y und der Index m die Bedeutungen haben, die bereits vorzugsweise für diese Substituenten und diesen Index genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie p-Toluolsulfonsäure und 1,5-Naphthalindisulfonsäure sowie Saccharin und Thiosaccharin.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen substituierten Azolylmethyloxiranen der Formel (I), in denen die Substituenten $R^1$, $R^2$, X und Y und der Index m die Bedeutungen haben, die bereits vorzugsweise für diese Substituenten und diesen Index genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, Salpetersäure und Schwefelsäure.

Als Beispiele für besonders bevorzugte erfindungsgemäße Stoffe der Formel (I) seien die in der folgenden Tabelle 1 aufgeführten Azolylmethyloxirane genannt.

Tabelle 1:

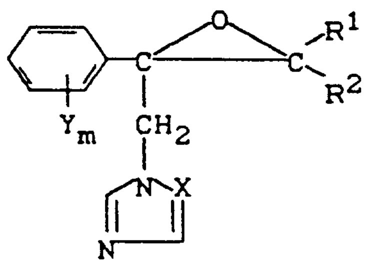

(I)

| $Y_m$ | $R^1$ | $R^2$ | X |
|---|---|---|---|
| 2,4-$F_2$ | $CH_3$ | $CH_3$ | N (CH) |
| 2-F, 4-$CF_3$ | $CH_3$ | $CH_3$ | " |
| 2-F, 4-$OCF_3$ | $CH_3$ | $CH_3$ | " |
| 2-F, 4-$SCF_3$ | $CH_3$ | $CH_3$ | " |
| 4-$OCF_3$ | $CH_3$ | $CH_3$ | " |
| 4-$SCF_3$ | $CH_3$ | $CH_3$ | " |
| 4-F | $CH_3$ | $CH_3$ | " |
| 3-F | $CH_3$ | $CH_3$ | " |
| 2-F | $CH_3$ | $CH_3$ | " |
| 2-Cl, 4-F | $CH_3$ | $CH_3$ | " |
| 2-F, 4-Cl | $CH_3$ | $CH_3$ | " |
| 3-Cl, 4-$OCF_3$ | $CH_3$ | $CH_3$ | " |
| 3-Cl, 4-$SCF_3$ | $CH_3$ | $CH_3$ | " |

Tabelle 1: (Fortsetzung)

| $Y_m$ | $R^1$ | $R^2$ | X |
|---|---|---|---|
| 3,4-$Cl_2$ | $CH_3$ | $CH_3$ | N (CH) |
| 2,4-$Cl_2$ | $CH_3$ | $CH_3$ | " |
| 3,4,6-$Cl_3$ | $CH_3$ | $CH_3$ | " |
| 3,4,6-$F_3$ | $CH_3$ | $CH_3$ | " |
| 3-F, 4,6-$Cl_2$ | $CH_3$ | $CH_3$ | " |
| 4-Br | $CH_3$ | $CH_3$ | " |
| 2-Br | $CH_3$ | $CH_3$ | " |
| 4-$OCH_3$ | $CH_3$ | $CH_3$ | " |
| 4- 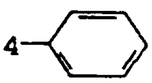 | $CH_3$ | $CH_3$ | " |
| 4- 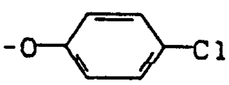 | $CH_3$ | $CH_3$ | " |
| 4-$CH_3$ | $CH_3$ | $CH_3$ | " |
| 4- 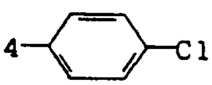 | $CH_3$ | $CH_3$ | " |
| 4- 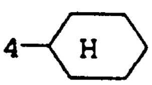 | $CH_3$ | $CH_3$ | " |
| 4-$SCH_3$ | $CH_3$ | $CH_3$ | " |
| 4-$OCHF_2$ | $CH_3$ | $CH_3$ | " |
| 4-$OCF_2Cl$ | $CH_3$ | $CH_3$ | " |
| 3-Cl, 4-$OCHF_2$ | $CH_3$ | $CH_3$ | " |
| 3-Cl, 4-$OCF_2Cl$ | $CH_3$ | $CH_3$ | " |
| 4-$SCHF_2$ | $CH_3$ | $CH_3$ | " |

Tabelle 1: (Fortsetzung)

| $Y_m$ | $R^1$ | $R^2$ | X |
|---|---|---|---|
| 2,4-$F_2$ | $CH_3$ | $C_2H_5$ | N (CH) |
| 2-F, 4-$CF_3$ | $CH_3$ | $C_2H_5$ | " |
| 2-F, 4-$OCF_3$ | $CH_3$ | $C_2H_5$ | " |
| 2-F, 4-$SCF_3$ | $CH_3$ | $C_2H_5$ | " |
| 4-$OCF_3$ | $CH_3$ | $C_2H_5$ | " |
| 4-$SCF_3$ | $CH_3$ | $C_2H_5$ | " |
| 4-F | $CH_3$ | $C_2H_5$ | " |
| 3-F | $CH_3$ | $C_2H_5$ | " |
| 2-F | $CH_3$ | $C_2H_5$ | " |
| 2-Cl, 4-F | $CH_3$ | $C_2H_5$ | " |
| 2-F, 4-Cl | $CH_3$ | $C_2H_5$ | " |
| 3-Cl, 4-$OCF_3$ | $CH_3$ | $C_2H_5$ | " |
| 3-Cl, 4-$SCF_3$ | $CH_3$ | $C_2H_5$ | " |
| 3,4-$Cl_2$ | $CH_3$ | $C_2H_5$ | " |
| 2,4-$Cl_2$ | $CH_3$ | $C_2H_5$ | " |
| 3,4,6-$Cl_3$ | $CH_3$ | $C_2H_5$ | " |
| 3,4,6-$F_3$ | $CH_3$ | $C_2H_5$ | " |
| 3-F, 4,6-$Cl_2$ | $CH_3$ | $C_2H_5$ | " |
| 4-Br | $CH_3$ | $C_2H_5$ | " |
| 2-Br | $CH_3$ | $C_2H_5$ | " |
| 4-$OCH_3$ | $CH_3$ | $C_2H_5$ | " |

Tabelle 1: (Fortsetzung)

| $Y_m$ | $R^1$ | $R^2$ | X |
|---|---|---|---|
| 4- 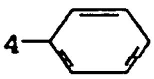 | $CH_3$ | $C_2H_5$ | N (CH) |
| 4- 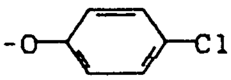 | $CH_3$ | $C_2H_5$ | " |
| 4-$CH_3$ | $CH_3$ | $C_2H_5$ | " |
| 4- 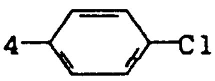 | $CH_3$ | $C_2H_5$ | " |
| 4- 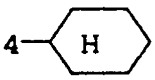 | $CH_3$ | $C_2H_5$ | " |
| 4-$SCH_3$ | $CH_3$ | $C_2H_5$ | " |
| 4-$OCHF_2$ | $CH_3$ | $C_2H_5$ | " |
| 4-$OCF_2Cl$ | $CH_3$ | $C_2H_5$ | " |
| 3-Cl, 4-$OCHF_2$ | $CH_3$ | $C_2H_5$ | " |
| 3-Cl, 4-$OCF_2Cl$ | $CH_3$ | $C_2H_5$ | " |
| 4-$SCHF_2$ | $CH_3$ | $C_2H_5$ | " |
| 4-Cl | $CH_3$ | $C_2H_5$ | " |
| 3-Cl | $CH_3$ | $C_2H_5$ | " |
| 2-Cl | $CH_3$ | $C_2H_5$ | " |
| 4-Cl | $C_2H_5$ | $C_2H_5$ | " |
| 3-Cl | $C_2H_5$ | $C_2H_5$ | " |
| 2-Cl | $C_2H_5$ | $C_2H_5$ | " |

Verwendet man beispielsweise 3,3-Dimethyl-3-phenoxy-2-phenyl-1-(1,2,4-triazol-1-yl)-2-propanol als Ausgangsstoff, Natriumhydrid als Base und Dioxan als Lösungsmittel, so kann der Ablauf des erfindungsgemäßen Verfahrens durch das folgende Formelschema wiedergegeben werden:

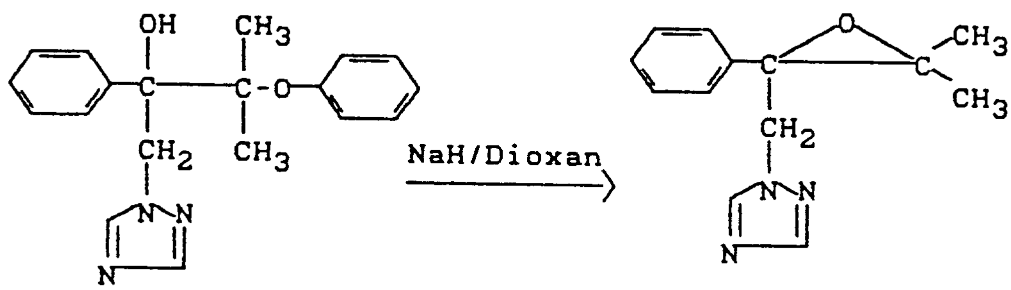


Die bei der Durchführung des erfindungsgemäßen Verfahrens als Ausgangsstoffe benötigten Hydroxyalkylazole sind durch die Formel (II) allgemein definiert. In dieser Formel haben $R^1$, $R^2$, X, Y und m vorzugsweise diejenigen Bedeutungen, die bereits im Zusammenhang mit der Beschreibung der erfindungsgemäßen Stoffe der Formel (I) vorzugsweise für diese Reste und diesen Index genannt wurden.

Die Hydroxyalkylazole der Formel (II) sind bekannt oder können nach literaturbekannten Verfahren hergestellt werden (vgl. DE-A 32 45 504).

Als Verdünnungsmittel können bei der Durchführung des erfindungsgemäßen Verfahrens alle üblichen inerten organischen Solventien eingesetzt werden. Vorzugsweise verwendbar sind Ether, wie Tetrahydrofuran und Dioxan; aromatische Kohlenwasserstoffe, wie Toluol und Xylol und aliphatische und cycloaliphatische Kohlenwasserstoffe, wie Hexan und Cyclohexan.

Als Basen können bei der Durchführung des erfindungsgemäßen Verfahrens alle üblicherweise für derartige Umsetzungen verwendbaren starken Basen eingesetzt werden. Vorzugsweise in Frage kommen Alkalimetallhydroxide wie z.B. Natrium- und Kaliumhydroxid, Alkalimetallalkoholate wie Natrium- und Kaliummethylat, -ethylat, -n-propylat und t-butylat, sowie Hydride wie zum Beispiel Natriumhydrid.

Die Reaktionstemperaturen können bei der Durchführung des erfindungsgemäßen Verfahrens in einem größeren Bereich variiert werden. Im allgemeinen arbeitet man bei Temperaturen zwischen 0 °C und 150 °C, vorzugsweise zwischen 20 °C und 100 °C. Die Reaktionszeit beträgt im allgemeinen 2 bis 24 Stunden, vorzugsweise 2 bis 10 Stunden.

Bei der Durchführung des erfindungsgemäßen Verfahrens setzt man auf 1 Mol Hydroxyalkylazol der Formel (II) im allgemeinen 1 bis 2 Mol, vorzugsweise 1 bis 1,2 Mol Base ein.

Die Isolierung der Verbindungen der Formel (I) erfolgt nach üblichen Methoden. Im allgemeinen geht man so vor, daß man das Reaktionsgemisch mit Wasser versetzt, dann mit einem mit Wasser wenig mischbaren organischen Lösungsmittel extrahiert und die vereinigten organischen Phasen nach gegebenenfalls vorherigem Waschen mit Wasser einengt. Das anfallende Produkt kann gegebenenfalls nach üblichen Methoden, zum Beispiel auf chromatographischem Wege, gereinigt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits weiter oben im Zusammenhang mit der Beschreibung der erfindungsgemäßen Metallsalz-Komplexe als bevorzugte Metallsalze genannt wurden.

Die Metallsalz-Komplexe von Verbindungen der Formel (I) können in einfacher Weise nach üblichen Verfahren erhalten werden, so z.B. durch Lösen des Metallsalzes in Alkohol, z.B. Ethanol, und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe eignen sich vorzugsweise als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Pythium-Arten, wie beispielsweise Pythium ultimum; Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg protektiv zur Bekämpfung von Sphaerotheca-Arten bei Gurken und Leptosphaeria-Arten bei Weizen einsetzen. Außerdem besitzen die erfindungsgemäßen Wirkstoffe auch gute fungizide Wirkung gegen Bohnenrost, Apfelschorf, Pyricularia an Reis sowie gegen Getreidekrankheitserreger wie Fusarium, Erysiphe, Puccinia und Cochliobolus.

Darüberhinaus greifen die erfindungsgemäßen Wirkstoffe in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten

kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Besonders geeignet sind die erfindungsgemäßen Wirkstoffe zur Hemmung des Wachstums bei Getreide, Sojabohnen, Baumwolle und Zuckerrüben.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser, Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid, Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide, Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalo cyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02 %, am Wirkungsort erforderlich.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg an Wirkstoff.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Die Herstellung und die Verwendung der erfindungsgemäßen Wirkstoffe geht aus den nachfolgenden Beispielen hervor.

Herstellungsbeispiele

Beispiel 1

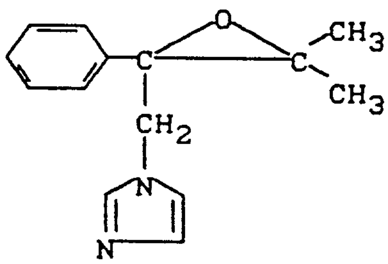

9 g (0,0253 Mol) 3,3-Dimethyl-3-(4-chlorphenoxy)-2-phenyl-1-(imidazol-1-yl)-2-propanol werden bei Raumtemperatur in 50 ml Dioxan gelöst, Man gibt 0,8 g (0,027 Mol) Natriumhydrid in das Gemisch, läßt 10 Minuten nachrühren, erwärmt langsam auf Rückflußtemperatur und läßt 10 Stunden nachrühren. Danach läßt man das Reaktionsgemisch auf Raumtemperatur abkühlen, gießt auf Wasser, extrahiert mit Dichlormethan und engt die organische Phase unter vermindertem Druck ein. Der Rückstand wird an Kieselgel mit einem Gemisch aus Essigester/Cyclohexan = 3:1 als Laufmittel chromatographiert.
Man erhält 4,0 g (69,4 % der Theorie) an 3,3-Dimethyl-2-phenyl-2-(imidazol-1-yl-methyl)-oxiran als Öl [$^1$H-NMR* : δ(1,01 (s)3H; 1,65 (s)3H)].

In analoger Weise zu Beispiel 1 und unter Berücksichtigung der Angaben zu dem erfindungsgemäßen Verfahren, werden die in der folgenden Tabelle 2 aufgeführten Verbindungen der Formel (I) hergestellt:

<u>Tabelle 2:</u>

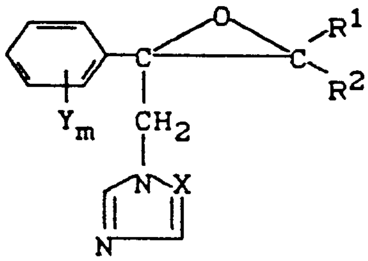

(I)

| Bsp. Nr. | $R^1$ | $R^2$ | X | 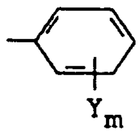 | physikalische Konstanten |
|---|---|---|---|---|---|
| 2 | $CH_3$ | $CH_3$ | N | 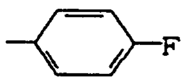 | Fp: 74° C |
| 3 | $CH_3$ | $CH_3$ | N | 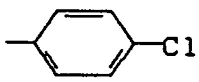 | $^1H$-NMR*): δ = 1,60(s) 3H; 0,93 (s)3H |
| 4 | $CH_3$ | $CH_3$ | N | 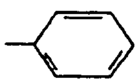 | $^1H$-NMR*): δ = 1,71(s) 3H; 1,05(s) 3H |
| 5 | $CH_3$ | $CH_3$ | N | 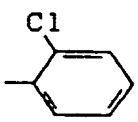 | $^1H$-NMR*): δ = 1,59(s) 3H; 1,01(s) 3H |
| 6 | $CH_3$ | $CH_3$ | N | 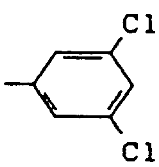 | $^1H$-NMR*): δ = 1,69(s) 3H; 1,08(s) 3H |

Tabelle 2 (Fortsetzung):

| Bsp. Nr. | $R^1$ | $R^2$ | X | 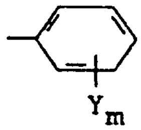 | physikalische Konstanten |
|---|---|---|---|---|---|
| 7 | $CH_3$ | $CH_3$ | N | 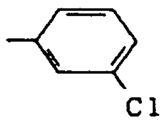 | $^1$H-NMR*): δ = 1,70(s) 3H; 1,06(s) 3H |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Verwendungsbeispiele

In den folgenden Verwendungsbeispielen werden die nachstehend angegebenen Verbindungen als Vergleichssubstanzen eingesetzt:

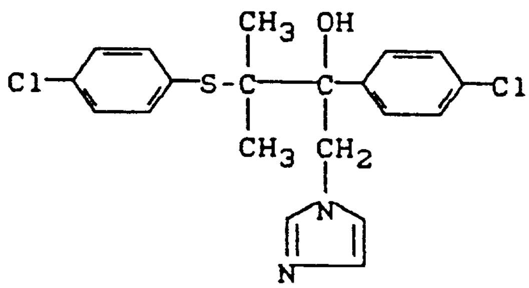

(A)

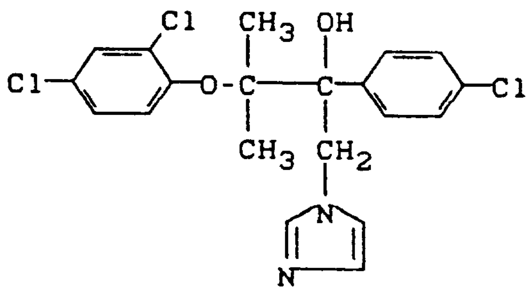

(B)

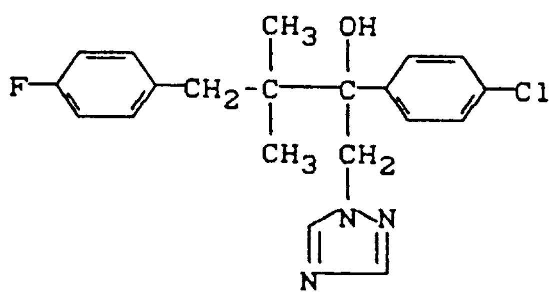

(C)

Die Verbindungen (A), (B) und (C) sind bekannt aus DE-A 32 45 504.

Beispiel A

Sphaerotheca-Test (Gurke) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24 °C und bei einer relativen Luftfeuchtigkeit von ca. 75 % im Gewächshaus aufgestellt.

10 Tage nach der Inokulation erfolgt die Auswertung.

In diesem Test zeigen die in den Beispielen (3), (5) und (7) aufgeführten erfindungsgemäßen Verbindungen eine wesentlich bessere Wirksamkeit als die Vergleichssubstanzen (A) und (B).

Beispiel B

Leptosphaeria nodorum-Test (Weizen)/protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 15 °C und einer relativen Luftfeuchtigkeit von ca. 80 % aufgestellt.

In diesem Test zeigen die in den Beispielen (1) und (4) aufgeführten erfindungsgemäßen Verbindungen eine wesentlich bessere Wirksamkeit als die Vergleichssubstanz (C).

Beispiel C

Wachstum bei Sojabohnen

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnaß mit den Wirkstoffzubereitungen besprüht. Nach 2 Wochen wird bei allen Pflanzen der Zuwachs gemessen und das Wachstum in Prozent des Zuwachses der Kontrollpflanzen berechnet. Es bedeuten 100 % Wachstum ein Wachstum entsprechend dem der Kontrollpflanzen und 0 % den Stillstand des Wachstums. Werte über 100 % kennzeichnen eine Wuchsförderung.

In diesem Test zeigt der erfindungsgemäße Wirkstoff (3) eine starke Wuchshemmung.

Beispiel D

Wachstum bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 2 Wochen wird der Zuwachs der Pflanzen gemessen und die Wuchshemmung in Prozent des Wachstum der Kontrollpflanzen berechnet. Es bedeuten 100 % Wachstum ein Wachstum entsprechend dem der Kontrollpflanzen und 0 % den Stillstand des Wachstums. Werte über 100 % kennzeichnen eine Wuchsförderung.

In diesem Test zeigt der erfindungsgemäße Wirkstoff (3) eine starke Wuchshemmung.

Beispiel E

Wachstum bei Zuckerrüben

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnaß mit den Wirkstoffzubereitungen besprüht. Nach 14 Tagen wird der Zuwachs der Pflanzen gemessen und das Wachstum in Prozent des Zuwachses der Kontrollpflanzen berechnet. Es bedeuten 100 % Wachstum ein Wachstum entsprechend dem der Kontrollpflanzen und 0 % den Stillstand des Wachstums. Werte über 100 % kennzeichnen eine Wuchsförderung.

In diesem Test zeigt der erfindungsgemäße Wirkstoff (3) eine starke Wuchshemmung.

**Patentansprüche**

1. Substituierte Azolylmethyloxirane der Formel

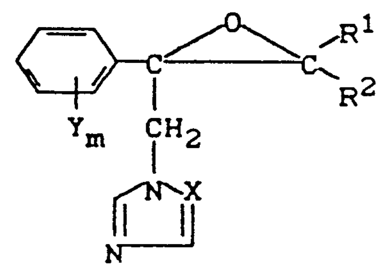

(I)

in welcher

- $R^1$ für geradkettiges oder verzweigtes Alkyl mit 1 bis 4 Kohlenstoffatomen steht,
- $R^2$ für geradkettiges oder verzweigtes Alkyl mit 1 bis 4 Kohlenstoffatomen steht,
- X für ein Stickstoffatom oder die CH-Gruppe steht,
- Y für Halogen, Alkyl mit 1 bis 4 Kohlenstoffatomen, Cycloalkyl mit 3 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, sowie für gegebenenfalls durch Halogen und/oder Alkyl mit 1 oder 2 Kohlenstoffatomen substituiertes Phenyl, gegebenenfalls durch Halogen und/oder Alkyl mit 1 oder 2 Kohlenstoffatomen substituiertes Phenoxy, gegebenenfalls durch Halogen und/oder Alkyl mit 1 oder 2 Kohlenstoffatomen substituiertes Phenylalkyl mit 1 oder 2 Kohlenstoffatomen im Alkylteil und/oder für gegebenenfalls durch Halogen und/oder Alkyl mit 1 oder 2 Kohlenstoffatomen substituiertes Phenylalkoxy mit 1 oder 2 Kohlenstoffatomen im Alkoxyteil steht, und
- m für die Zahlen 0, 1, 2 oder 3 steht,

wobei die Bedeutungen von Y gleich oder verschieden sein können, wenn m für die Zahlen 2 oder 3 steht,
sowie deren Säureadditions-Salze und Metallsalz-Komplexe.

2. Verfahren zur Herstellung von substituierten Azolylmethyloxiranen der Formel (I) gemäß Anspruch 1 sowie von deren Säureadditions-Salzen und Metallsalz-Komplexen, dadurch gekennzeichnet, daß man Hydroxyalkylazole der Formel

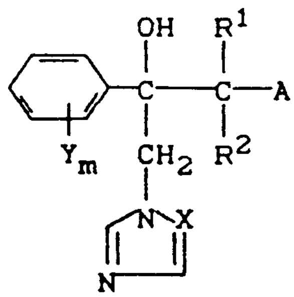

(II)

in welcher

- $R^1$, $R^2$, X, Y und m die oben angegebene Bedeutung haben und
- A für einen Phenolat-, 4-Chlorphenolat- oder Thiophenolat-Rest steht,

gegebenenfalls in Gegenwart eines Verdünnungsmittels mit starken Basen umsetzt und gegebenenfalls anschließend an die so erhaltenen Verbindungen der Formel (I) eine Säure oder ein Metallsalz addiert.

3. Fungizide und pflanzenwuchsregulierende Mittel, gekennzeichnet durch einen Gehalt an mindestens einem substituierten Azolylmethyloxiran der Formel (I) gemäß Anspruch 1 bzw. an einem Säureadditions-Salz oder Metallsalz-Komplex eines substituierten Azolylmethyloxirans der Formel (I).

4. Verwendung von substituierten Azolylmethyloxiranen der Formel (I) gemäß Anspruch 1 bzw. von deren Säureadditions-Salzen und Metallsalz-Komplexen zur Bekämpfung von Pilzen, ausgenommen die Behandlung des menschlichen und tierischen Körpers, sowie zur Regulierung des Pflanzenwachstums.

5. Verfahren zur Bekämpfung von Pilzen sowie zur Regulierung des Pflanzenwachstums, dadurch gekennzeichnet, daß man substituierte Azolylmethyloxirane der Formel (I) gemäß Anspruch 1 bzw. deren Säureadditions-Salze oder Metallsalz-Komplexe auf die Pflanzen und/oder deren Lebensraum ausbringt.

6. Verfahren zur Herstellung von fungiziden und pflanzenwuchsregulierenden Mitteln, dadurch gekennzeichnet, daß man substituierte Azolylmethyloxirane der Formel (I) gemäß Anspruch 1 bzw. deren Säureadditions-Salze oder Metallsalz-Komplexe mit Streckmitteln und/oder oberflächenaktiven Stoffen vermischt.

## Claims

1. Substituted azolylmethyloxiranes of the formula

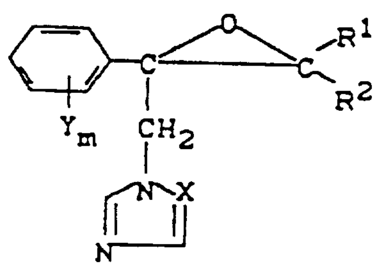

(I)

in which

- $R^1$ represents straight-chain or branched alkyl having 1 to 4 carbon atoms,
- $R^2$ represents straight-chain or branched alkyl having 1 to 4 carbon atoms,
- X represents a nitrogen atom or the CH group,
- Y represents halogen, alkyl having 1 to 4 carbon atoms, cycloalkyl having 3 to 7 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, and also represents phenyl which is optionally substituted by halogen and/or alkyl having 1 or 2 carbon atoms, phenoxy which is optionally substituted by halogen and/or alkyl having 1 or 2 carbon atoms, phenylalkyl which has 1 or 2 carbon atoms in the alkyl moiety and which is optionally substituted by halogen and/or alkyl having 1 or 2 carbon atoms and/or represents phenylalkoxy which has 1 or 2 carbon atoms in the alkoxy moiety and which is optionally substituted by halogen and/or alkyl having 1 or 2 carbon atoms, and
- m represents the numbers 0, 1, 2 or 3,

it being possible for the meanings of Y to be identical or different when m represents the numbers 2 or 3,
and their acid addition salts and metal salt complexes.

2. Process for the preparation of substituted azolylmethyloxiranes of the formula (I) according to Claim 1 and of their acid addition salts and metal salt complexes,
characterized in that hydroxyalkylazoles of the formula

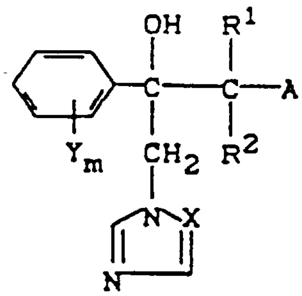

(II)

in which

R[1], R[2], X, Y and m have the abovementioned meaning and

A represents a phenolate, 4-chlorophenolate or thiophenolate radical

are reacted with strong bases, if appropriate in the presence of a diluent, and the resulting compounds of the formula (I) are then, if appropriate, subjected to an addition reaction with an acid or a metal salt.

3. Fungicidal and plant growth-regulating agents, characterized in that they contain at least one substituted azolylmethyloxirane of the formula (I) according to Claim 1 or of an acid addition salt or metal salt complex of a substituted azolylmethyloxirane of the formula (I).

4. Use of substituted azolylmethyloxiranes of the formula (I) according to Claim 1 or of their acid addition salts and metal salt complexes for combating fungi, with the exception of the treatment of the human and animal body, and for regulating plant growth.

5. Method of combating fungi and of regulating plant growth, characterized in that substituted azolylmethyloxiranes of the formula (I) according to Claim 1 or their acid addition salts or metal salt complexes are applied to the plants and/or their environment.

6. Process for the preparation of fungicidal and plant growth-regulating agents, characterized in that substituted azolylmethyloxiranes of the formula (I) according to Claim 1 or their acid addition salts or metal salt complexes are mixed with extenders and/or surface-active substances.

**Revendications**

1. Azolylméthyloxirannes substitués de la formule

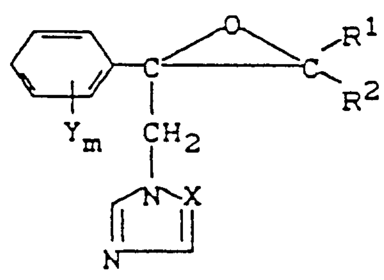

(I)

dans laquelle

$R^1$ représente un radical alkyle en chaîne droite ou ramifiée avec 1 à 4 atomes de carbone,

$R^2$ représente un radical alkyle en chaîne droite ou ramifiée avec 1 à 4 atomes de carbone,

X représente un atome d'azote ou un radical CH,

Y représente un halogène, un radical alkyle avec 1 à 4 atomes de carbone, cycloalkyle avec 3 à 7 atomes de carbone, alcoxy avec 1 à 4 atomes de carbone, alkylthio avec 1 à 4 atomes de carbone, alkyle halogéné avec 1 ou 2 atomes de carbone et 1 à 5 atomes d'halogène identiques ou différents, alcoxy halogéné avec 1 ou 2 atomes de carbone et 1 à 5 atomes d'halogène identiques ou différents, alkylthio halogéné avec 1 ou 2 atomes de carbone et 1 à 5 atomes d'halogène identiques ou différents, aussi bien que phényle éventuellement substitué par un halogène et/ou un radical alkyle avec 1 ou 2

atomes de carbone, phénoxy éventuellement substitué par un halogène et/ou un radical alkyle avec 1 ou 2 atomes de carbone, phénylalkyle éventuellement substitué par un halogène et/ou un radical alkyle avec 1 ou 2 atomes de carbone, avec 1 ou 2 atomes de carbone dans la partie alkyle et/ou phénylalcoxy éventuellement substitué par un halogène et/ou un radical alkyle avec 1 ou 2 atomes de carbone, avec 1 ou 2 atomes de carbone dans la partie alcoxy, et

m représente le nombre 0, 1, 2 ou 3,

où les significations de Y peuvent être identiques ou différentes quand m représente le nombre 2 ou 3,

aussi bien que leurs sels d'addition d'acides et leurs complexes de sels métalliques.

2. Procédé de préparation des azolylméthyloxirannes substitués de la formule (I) selon la revendication 1, aussi bien que de leurs sels d'addition d'acides et de leurs complexes des sels métalliques, caractérisé en ce que des hydroxyalkylazoles de la formule

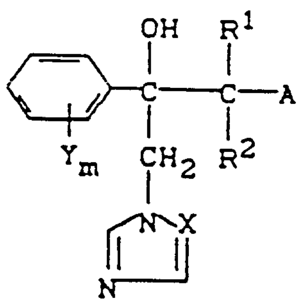

(II)

dans laquelle

$R^1$, $R^2$, X, Y et m ont les significations données ci-dessus et A représente un reste de phénolate, de 4-chlorophénolate ou de thiophénolate, éventuellement en présence d'un diluant, sont mis en réaction avec des bases fortes et éventuellement, ensuite, un acide ou un sel métallique est ajouté au composé de la formule (I) ainsi obtenu.

3. Fongicide et régulateur de croissance des plantes, caractérisé en ce qu'il contient au moins un azolylméthyloxiranne substitué de la formule (I) selon la revendication 1 ou bien un sel d'addition d'acides ou un complexe de sel métallique d'un azolylméthyloxiranne substitué de la formule (I).

4. Utilisation des azolylméthyloxirannes substitués de la formule (I) selon la revendication 1 ou bien d'un de leurs sels d'addition d'acides ou d'un de leurs complexes de sels métalliques pour lutter contre les champignons, à l'exception du traitement des corps humains ou animaux, aussi bien que pour contrôler la croissance des plantes.

5. Procédé pour lutter contre les champignons aussi bien que pour contrôler la croissance des plantes, caractérisé en ce qu'on utilise des azolylméthyloxirannes substitués de la formule (I) selon la revendication 1 ou bien un de leurs sels d'addition d'acides ou un de leurs complexes de sels métalliques sur les plantes et/ou leur habitat.

6. Procédé de préparation de fongicides et de régulateurs de croissance, caractérisé en ce qu'on mélange des azolylméthyloxirannes substitués de la formule (I) selon la revendication 1 ou bien un de leurs sels d'addition d'acides ou un de leurs complexes de sels métalliques avec des diluants et/ou des agents tensioactifs.